# EUROPEAN PATENT APPLICATION

(11) **EP 1 916 018 A1**
(43) Date of publication of application: **30.04.2008**
(21) Application number: 06076920.5
(22) Date of filing: 24.10.2006
(51) Int. Cl.: A61N 5/10, A61B 18/18

(54) **Pulsed radiofrequency for intra-articular therapy of pain**

(71) Applicant: Cotop International B.V., 1033 RW Amsterdam (NL)
(72) Inventor: Sluijter, Menno Emanuel, 6005 Luzern (CH); Teixeira, Alexandre Jose Leonardo, Porto, 4100-376 (PT)
(74) Representative: van Loon, C.J.J.

(57) **Abstract**

The invention provides for a method of treating pain in a joint of a subject with pulsed radiofrequency. To enable such a treatment a specific electrode is provided comprising a completely insulated outer needle with a sharp tip, a removable stylet, and one or more conductive, blunt ended, optionally flexible electrodes, optionally with an insulated tip.

## Description

The invention relates to the field of medicine, more specifically to the field of anodynia, more specifically to the field of pain relief by treatment with radiofrequency electrical fields.

The first reports of the use of electrical current for pain management appeared in the 1930's. Initially, DC was used to induce lesions of nerves through the temperature increase caused by the electrical current (thermocoagulation). Later, this has been replaced by AC with a frequency of 400 to 500,000 Hz, which has been shown to deliver more precise lesions. In the past few decades this radiofrequency (RF) thermocoagulation has been established as an accepted treatment option for trigeminal neuralgia, for unilateral cancer pain and for zygoapophyseal joint pain. Further, RF has been used in other fields:
- in cardiology for thermocoagulation of nerves in the heart that conduct aberrant stimulus patterns;
- in oncology for destroying tumor tissue;
- in orthopedics for treatments of cartilage defects in arthrosis.

The application in other pain syndromes (e.g. acute and chronic radicular pain, peripherial neuralgias) has been limited due to the fear of producing deafferentiation pain. The development of a novel method for administrating high frequency current, pulsed radiofrequency (PRF) allowed using it to treat other pathologies and nerve structures. With PRF, current is delivered in pulses of short duration (1-100 msec) separated by a silent period of about 0,1 to 1 sec. Output current is set not to exceed 42°C to prevent neurodestruction. Heat generated by the application of the current is dissipated between pulses. Nowadays PRF is recognised as treatment for e.g. various forms of spinal and facial pain and peripheral neuralgias.

The electrode, which delivers the current at the tissue should be accurately placed in the near vicinity of the nerve to be treated. This is necessary because the electrical field evoked at the electrode tip rapidly loses strength at increasing distances from the tip. This is not a problem when the pain is caused by, or conducted by one single nerve or nerve structure. However, when pain is caused by pathology of a joint, this is one of the major drawbacks of the current method, because many joints are innervated by multiple nerves, originating from multiple spinal levels.

### SUMMARY OF THE INVENTION

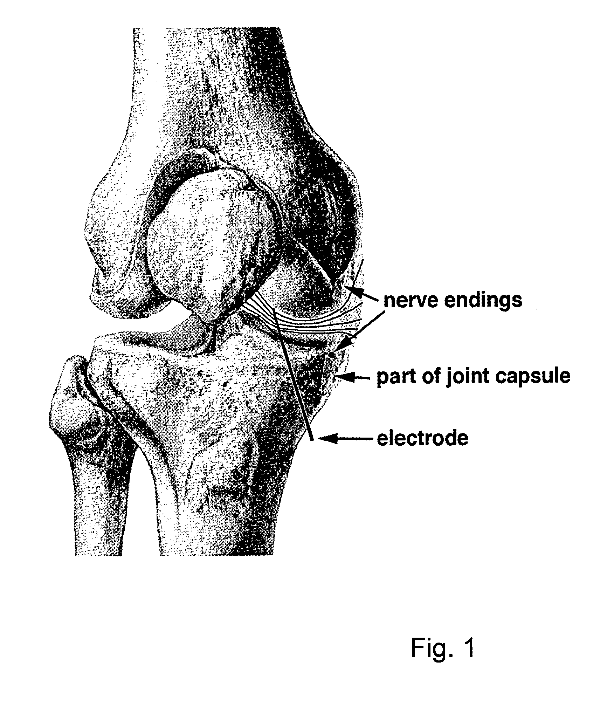

The invention now is related to the treatment of chronic pain emanating from joints by intra-articular application of pulsed radiofrequency (PRF) electrical current. At first sight it is surprising that such a treatment is possible, because many of the nociceptor nerve endings that are responsible for pain sensation are located in the joint capsule, at considerable distance from the electrode tip.. An electrical field as produced in PRF would thus be held to be extinguished before the majority of the nociceptors are reached. Apparently, however, the bony structures surrounding the joint function as insulator, and cause the electric field to maintain sufficient strength over larger distances, at least sufficient to exert its pain alleviating effect (fig. 1).

Therefore the invention comprises a method for treatment of pain in a joint of a subject comprising the steps of:
a) placing at least one electrode with the tip in the cavity of the affected joint; and
b) applying pulsed radio frequency (PRF) electrical current through the at least one electrode.

Preferably in such a method the subject is a human being, but it may also be an animal, and the joint is a wrist, an elbow, a shoulder, a knee, a hip, an ankle, the sacro-iliacal joint, the small joints of hand and foot, the zygoapophyseal joints, the lateral atlantoaxial joint or the atlantooccipital joint, or any other joint in the body.

The invention further provides an electrode assembly for use in PRF therapy for joint pain, comprising:
a) a hollow needle, with a sharp tip, which is completely insulated
b) a removable stylet; and
c) a conductive, blunt ended, optionally flexible electrode, optionally with an insulated tip.
Said electrode assembly may or may not comprise a thermocouple.

Also provided by the invention is a system for intra-articular application of PRF comprising:
a) an electrode assembly as defined above;
b) a PRF current generator, that is specially adapted to the intra-articular application of PRF, optionally with multiple current outlets with an asynchronous duty cycle; and
c) means for connecting a) and b) and means to connect a patient to earth.

### LEGENDS TO THE FIGURES

Fig. 1. Schematic drawing of a knee joint and part of the joint capsule, containing the nerve endings. The solid lines indicate the electric field lines.

Fig. 2a. Diagram of an outer needle containing a stylet according to the invention. The needle (thick black line) is completely insulated at the outside.

Fig. 2b. Diagram showing the needle with an extended inner electrode part. After removal of the stylet from the needle of Fig. 1a, a flexible electrode part is inserted in the needle and extending from it. Note the blunt end of the electrode for preventing damage to the tissue in which it is inserted.

Fig. 3a. Diagram of an outer needle with a 6° bend of the distal part, forcing the electrode to come out at a slight angle.

Fig. 3b. Diagram of an outer needle with a side outlet. The electrode comes out at a larger angle, facilitating treatment of a joint that cannot be approached with a straight needle.

Fig. 3c. Diagram showing an electrode with an insulated tip.

Fig. 4 Example of a catheter with multiple electrodes positioned in the hip joint.

### DETAILED DESCRIPTION

PRF (pulsed radiofrequency) is a clinically proven method to alleviate pain in cases where pain sensation is due to or transported via peripheral nerves (such as in case of pain caused by pinching a nerve by a slipped disc of the spinal column, facial pain, trauma, etc.). As discussed above, PRF, just like RF, works through applying an electrical AC current to the vicinity of a nerve. Usually a frequency of 400.000 - 500.000 Hz is used, but the range may vary from 50.000 to 1.000.000 HzIn PRF, in contrast to continuous RF, the heat that is generated at the tip of the electrode during the active phase of the duty cycle is dissipated during the resting phase of zero, or of appreciably lower voltage. The settings of the current generator should be adjusted so that the mean temperature around the electrode tip does not rise to neurodestructive levels, which start from 45°C upwards. It is allowable that the temperature may briefly rise above 45° C during the active phase of the duty cycle (the so-called heat spikes), although the biological effects of these ultrashort rises in temperature are not known. However, the spread of heat into the tissues during a heat spike has been predicted to be minimal (< 0.2 mm), thereby outruling that a thermal (coagulation or neurotomy) effect is the cause of the clinical efficacy of PRF treatment - at the same time also casting doubts whether the thermal effect would be crucial for RF. As for yet, there is no conclusive theory explaining and supporting the observed effects of PRF.

PRF (and RF) have been shown to be only effective if the electrical current is applied near the nerve to be treated. This is understandable, since the current density, and therefore the electric field, will rapidly fall off as the distance to the electrode increases. A field around an electrode can be thought of as a spherical field ahead of the electrode tip and a cylindrical field around its shaft. The strength of the field will be inversely proportional to d² ahead of the electrode, and inversely proportional to d alongside the shaft in which d is the distance from the tip. The same will apply to the heat generated in RF. The tissue in which a biologically active electric field is generated will thus be small (about 2 - 5 mm for a voltage of 45 Volts).

In cases where pain is caused or transported by a network of many- mostly smaller - nerves, recently it has been proposed by O'Keeffe et al. (US 2006/0025832 and US 2006/0030899) to apply a multiplicity of electrodes for delivering the current to the affected region. However, each of those electrodes would suffer from the same decrease of the electrical field, and the general effect would only be obtainable by placing sufficient electrodes in the affected region.

Many people experience pain in the joints. Especially the case of pain in the joints by trauma, a bad posture and/or obesitas or in disorders like whiplash, rheumatoid arthritis and arthrosis, the pain in one or more joints can be the cause of a decreased quality of life. These joint pains share that there is no one, single peripheral nerve which is responsible for the cause or transport of the pain stimuli, because the innervation of joints in many cases is complex. Thus, this would make treatment with RF or PRF cumbersome or impossible. Nevertheless, RF of innervating nerves has been applied frequently for joint pains, especially in the case of spinal pains, like sacroiliac joint pain or low back pain of zygoapophysealjoint origin. However, according to a recent systematic review of the literature on these applications ("Radiofrequency Neurotomy as Treatment for Spinal Joint Pain: A Systematic Review of the Literature", April 2006, Monash University, Australia) there is no consistent or strong evidence that RF is efficacious in the treatment of spinal joint pain.

As for intra-articular application of continuous RF, this possibility has been described by Sanders (The Pain Clinic, 1998, 10:253-59) However, this method has never been followed by others since it causes serious damage to the joint. It is well known that chondrocytes are irreversibly damaged by temperatures of 45°C and up.

Yet, despite the varying and inconclusive results with RF treatment of joint pain, it has now been discovered that PRF gives good results when applied intra-articularly.

The instruments for applying PRF to a patient comprise a needle-like electrode, connected to a PRF current source and a means for providing connection to earth. The PRF current source (or lesion-generator) will provide, next to the source for the current, also a stimulator function, to check for the proper positioning of the electrode, and the facility of measuring the impedance of the circuit between patient, earth and apparatus. These devices are commercially available (e.g. the Cosman RFG-1B Radiofrequency Lesion Generator from Cotop International, the Netherlands). The procedure to apply PRF to a tissue would be to first guide the electrode to the place where the current should be delivered. Positioning of the electrode is usually guided by X-ray imaging and confirmed by electrical stimulation.

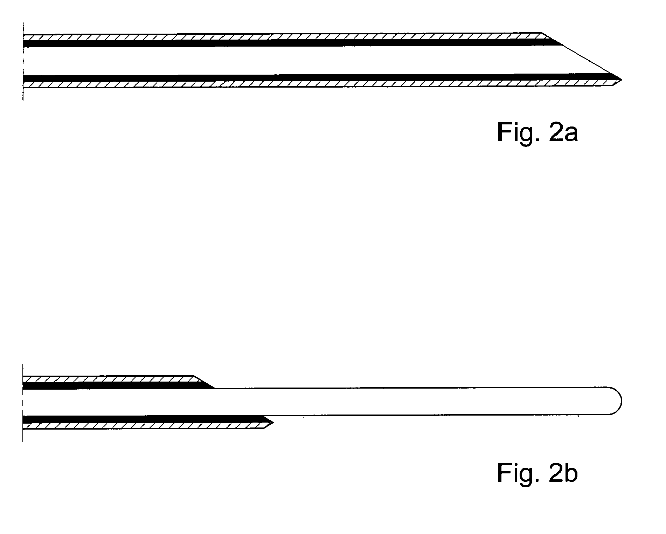

For intra-articular placement of the electrode, the electrodes which are currently used for (P)RF are unsuitable since they have a very sharp tip, which could damage the joint. Further, some joints are arcuated, which limits the use of rigid instruments. For this purpose, the invention also comprises a new electrode for use in intra-articular PRF. Basically such an electrode comprises a hollow outer (needle) part, initially provided with a releasable inner stylet and an inner, true electrode part. The outer part is completely insulated, and the insulation material may be any non-conductive material which does not react with biological tissue, such as plastics like polyethylene, polypropylene and the like. For ease of penetration through the skin and underneath tissues it has a very sharp tip. Also the stylet, which fits into the hollow needle is as sharp-edged as the needle (see Fig. 2a). After perforation of the capsule of the joint, the stylet is removed. The inner part, the actual electrode, is then inserted into the needle and will extend from the hollow outer needle into the joint (see Fig. 2b). The electrode has a blunt end and consists mainly of conductive material, such as metal or doped conductive polymers. Preferably, the electrode is flexible to allow it to follow the natural curves of the joint surface. The electrode optionally comprises a thermocouple for continuous measurement of the temperature at the tip.

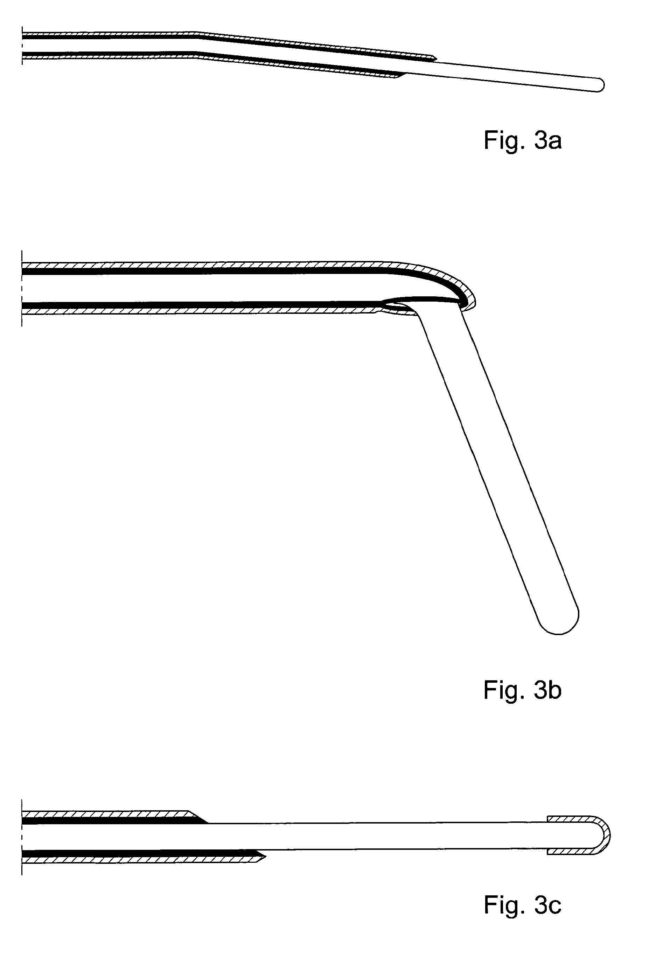

The outer needle may optionally be constructed in such a way that the electrode comes out at an angle. When the tip is slightly curved (fig. 3a), the electrode will come out at an angle of 5 - 10°. This would be advantageous to direct the electrode in a desired direction once the capsule of the joint has been perforated. When the needle is constructed with a side outlet the angle may be larger (fig. 3b). This option will have advantages when a straight approach to the joint is not possible for anatomical reasons.

Another option is insulation of the tip of the electrode (fig 3 c). It is around the tip of the electrode that the electric and thermal fields are strongest, and it has been shown that these strong fields may cause a microscopically small area of necrosis around a sharp needle tip. This effect is already minimalised by the fact that in this case the electrode is blunt, but since the tip will often lie very close to the articular surfaces it is worth taking any possible precaution. By insulating the tip any damage would be prevented or at least further reduced.

The dimensions of the electrode assembly (i.e. the hollow needle, stylet and actual electrode), such as the length, the inner and outer diameter, and the length of the electrode extending from the needle will be variable, depending on the size of the joint and the distance of the joint from the point of insertion of the needle on the skin. The diameter of the assembly will amongst others be determined by the presence of a thermocouple and the number of electrodes which will be guided through the needle.

At the proximal end, the needle, stylet and/or electrode will have internal or external means for fixing them together and/or to prevent the parts to dislocate. Such means can be projections which function to block movement or screw threads which can be used to screw parts together. The person skilled in the art will know further alternatives and how to provide them. The electrode may have appropriate markings at its proximal end so that the distance that the electrode extends beyond the tip of the needle can be measured.

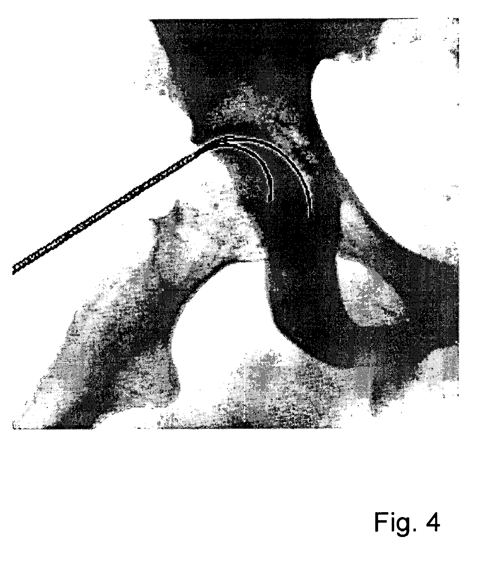

For larger joints, it is contemplated that one electrode will not be sufficient to provide the desired clinical effect. This problem can be solved by inserting multiple electrodes through one needle with a larger diameter, whereby the flexible electrodes can be manoeuvred to spread into the joint cavity. Further, if the joint cavity appears to be too arcuate for the flexible electrode, a thin, helical catheter made of conducting metal can be used as electrode (fig. 4).

When the electrode is in the proper position and connected to the PRF current source, the patient will be connected to earth (e.g. by a so-called earth-plate) to establish an electrical circuit. Exposure to PRF is then applied. Usual values are a pulse duration of 10 msec and a pulse frequency of 2/sec; a voltage of 20 - 80 V depending on the size of the joint and the thermal effects; and a total duration of treatment of 10 minutes. There is however a wide variation in parameters that may be used:
Frequency: 50.000 - 1.000.000 Hz, preferably 150.000 - 500.000 Hz
Pulse duration: 0.1 - 100 msec, preferably 5-20 msec.
Pulse frequency: 1 - 20/sec, preferably1 - 3/sec
Voltage: 10 - 80 V, preferably 40-60 V
Treatment time: 2 - 30 minutes

Further the duty cycle may be irregular, with varying pulse duration and pulse frequency, and the voltage may not be brought back to zero during the rest phase.

The RF Lesion Generators that are commercially available are suitable for performing this procedure. However, modifications would greatly facilitate this particular procedure. Since the total exposure times that have so far been used are long (10 - 20 min.) total procedure time will be unacceptably long if multiple joints have to be treated, such as is often the case when treating zygoapophyseal joints, or if it is preferred to treat two or more electrode positions in a large joint, such as the knee or the hip. In those cases it is advantageous to use a power source with multiple current outlets. Such instruments with multiple outlets are commerciably available, for example the lesion generator NeuroTherm™ NT100 (manufactured by RDG Medical, Croydon,United Kingdom). However, this instrument is made with the purpose of making simultaneous lesions with continuous RF in a number of nerves, e.g. a number of medial branches innervating the zygoapophyseal joints.

If two or more electrodes are placed in a joint and if these electrodes were powered by a power source with multiple current outlets, simultaneous firing of the outlets may interfere with the creation of an optimal electric field around each electrode. It is therefore essential that the outlets fire asynchronously.

The method of the invention and the electrode assembly and system of the invention can be applied to both animals and human beings. Preferably, the intra-articular application of PRF is performed on humans in need of such a therapy, but also larger animals, especially domestic animals like horses, dogs and cats, or zoo animals, like giraffes, zebras and the like, can be treated with PRF according to the invention.

The invention will now be illustrated by the following examples, which are illustrative only and are not intended to limit the scope of the invention.

### Example 1.

A 78 year old man complained since 4 years increasingly about L-sided continuous neck pain, with shooting pains superimposed. His pain intensity was 8 on a scale of 0 - 10. After 5 minutes walking he felt the shooting pain with every step and he could no longer perform work in his garden. On physical examination there was marked tenderness over the L side of the neck, and the X-rays showed a large, hypertrophied zygoapophyseal joint C3/C4 on the left side.

He was first treated with PRF treatment of the nerves to the zygoapophyseal joints at levels C3 to C5. This was unsuccessful. An electrode was then placed in the arthrotic joint, and PRF was applied at 2 x 10 msec/sec, at a voltage of 50 V, for 10 minutes. At checkup 2 months later he was 100% free of pain. He could walk unlimited distances and he worked the garden with a chain saw for hours.

### Example 2.

An 82 year old priest complained increasingly about pain in both knees, L>R. This impaired his walking range, but what bothered him most was that he no longer could kneel down to say his prayers. X-rays showed advanced arthrosis of both knees. An operation, with implant of a prosthesis was advised but since both knees were involved he worried about the consequences.

He underwent PRF treatment of the L knee. The electrode was placed in two locations, one on the medial and one on the lateral side. In both locations PRF was applied, 2 x 10 msec/sec, at 60 V for 10 minutes. He became free of pain in the L knee and he could kneel again to say his prayers.

## Claims

1. Method for treatment of pain in a joint of a subject comprising the steps of:
a) placing at least one electrode with the tip in the cavity of the affected joint; and
b) applying pulsed radio frequency (PRF) electrical current through the at least one electrode.

2. Method according to claim 1, wherein the subject is a human being.

3. Method according to claim 1, wherein the joint is a wrist, an elbow, a shoulder, a knee, a hip, an ankle, a spinal disc, the sacro-iliacal joint, the small joints of hand and foot, the zygoapophyseal joints, the lateral atlantoaxial joint or the atlantooccipital joint, or any other joint in the body.

4. Electrode assembly for use in PRF therapy for joint pain, comprising:
a) a hollow needle, with a sharp tip, which is completely insulated
b) a removable stylet; and
c) one or more conductive, blunt ended, optionally flexible electrodes.

5. Electrode assembly according to claim 4, wherein the tip of the outer needle is bent, or has a side outlet, so that the electrode comes out at an angle.

6. Electrode assembly according to claim 4 or 5, wherein the assembly further comprises a thermocouple.

7. Electrode assembly according to claim 4, 5 or 6, wherein the tip of the electrode is insulated

8. System for intra-articular application of PRF comprising:
a) an electrode assembly according to claim 4, 5, 6 or 7
b) a PRF current generator with multiple current outlets, wherein the pulses at the various outlets are delivered asynchronously
c) means for connecting a) and b) and means to connect a patient to earth.

9. Use of an electrode assembly according to claim 4, 5, 6, or 7 or a system according to claim 8 for intra-articular application of PRF.
